# FASCICULE DE BREVET EUROPEEN

(11) **EP 2 257 587 B1**
(45) Date de publication et mention de la délivrance du brevet: **12.06.2019**
(21) Numéro de dépôt: 09720962.1
(22) Date de dépôt: 04.03.2009
(51) Int. Cl.: C08G 73/00, C08G 73/02, C08L 79/00, C08L 79/02, A61K 47/00, A61K 48/00

(54) **POLYMERE DERIVE DE LA POLYETHYLENIMINE LINEAIRE POUR LE TRANSFERT DE GENE**
POLYMER AUS LINEAREM POLYETHYLENIMIN FÜR GENÜBERTRAGUNG
POLYMER DERIVED FROM LINEAR POLYETHYLENIMINE FOR GENE TRANSFER

(30) Priorité: 05.03.2008 FR 0851434
(43) Date de publication de la demande: 08.12.2010
(73) Titulaire: Centre National de la Recherche Scientifique (C.N.R.S.), 75016 Paris (FR); Universite d'Orleans, 45000 Orléans (FR); Universite d'Evry Val d'Essonne, 91000 Evry (FR)
(72) Inventeur: CHERADAME, Hervé, F-92200 Neuilly Sur Seine (FR); SASSATELLI, Mathieu, F-91610 Ballancourt Sur Essonne (FR); GUEGAN, Philippe, F-75012 Paris (FR); MIDOUX, Patrick, F-45500 Saint Denis De L'hotel (FR); PICHON, Chantal, F-45500 Saint Denis De L'hotel (FR)
(74) Mandataire: Regimbeau
(86) Numéro de dépôt international: PCT/EP2009/052530
(87) Numéro de publication internationale: WO 2009/112402

(56) Documents cités:
- US-A1- 2006 093 674
- US-B1- 6 372 499
- A.SWAMI, A.AGGARWAL, A.PATHAK, S.PATNAIK, P.KUMAR, Y.SINGH, K.C.GUPTA: "Imidazolyl-PEI modified nanoparticles for enhanced gene delivery" INTERNATIONAL JOURNAL OF PHARMACEUTICS, vol. 335, 2007, pages 180-192, XP002497143
- A.BENTOLILA, I.VLODAVSKY, R.IDHAI-MICHAELI, O.KOVALCHUK, C.HALOUN, A.J.DOMB: "Poly(N-acryl amino acids): a new class of biologically active polyanions" JOUNRAL OF MEDICAL CHEMISTRY, vol. 43, 2000, pages 2591-2600, XP002497142

## Description

L'invention concerne les polyéthylènimines et leurs utilisations dans le transfert de gène en vue d'applications thérapeutiques.

Dans ces applications, on cherche à corriger des défauts génétiques dans des cellules cibles. Le transfert de gène peut s'effectuer *in vitro, ex vivo* ou *in vivo.* Dans les opérations effectuées *in vitro* et *ex vivo,* on cherche à modifier le fonctionnement biologique de cellules au sein d'une culture, tandis que l'administration *in vivo* sur un animal ou un patient se propose d'agir directement sur des tissus ou organes cibles, afin de modifier les biosynthèses de protéine. Le principal problème auquel se heurte la technique de transfert de gène réside dans l'efficacité du transfert dans les tissus ou cellules visés, avec un haut niveau d'expression du gène souhaité. Le transfert de gène s'effectue à l'aide de complexes essentiellement constitués de deux parties. D'une part, l'élément actif est un acide nucléique, un plasmide, un fragment d'ADN ou d'ARN. D'autre part, une protection, pouvant aussi comporter des fonctions susceptibles d'aider à la localisation du complexe sur les cellules ou tissus cibles désirés, peut être également présente. On désigne généralement cette protection sous le nom de vecteur de transfert de gène, pour lequel on distingue deux types principaux, les vecteurs viraux et les vecteurs synthétiques (ou non viraux). C'est dans cette dernière catégorie que se situe la présente invention.

Les vecteurs non viraux se subdivisent à leur tour en deux classes principales, lipides cationiques ou vecteurs polymères. Quel que soit le vecteur, on recherche des formulations qui soient toujours plus efficaces, avec une toxicité la plus réduite possible, afin de pouvoir servir de base à un traitement thérapeutique. Les polyélectrolytes positifs constituent un ensemble de vecteurs polymères particulièrement adapté au transfert de gène, dans la mesure où leur charge positive leur confère des propriétés de complexation de l'ADN et des plasmides, et d'interaction avec les surfaces cellulaires visées. Le complexe résultant a des caractéristiques de taille et de charge de surface telles que son internalisation dans certaines cellules soit très favorisée. Parmi les polyélectrolytes positifs déjà utilisés pour le transfert de gène il faut citer la poly-L-lysine et ses dérivés, le chitosane et ses dérivés ainsi que des saccharides fonctionnalisés par des fonctions amine, le poly(méthacrylate de diméthylaminoéthyle), et la polyéthylènimine (PEI) et ses dérivés. L'invention concerne cette dernière classe de polyélectrolytes positifs à base de PEI.

Les poly(éthylènimine)s (PEI) ont été les premiers matériaux du type polyélectrolyte positif à avoir été introduits dans le domaine des vecteurs polymères cationiques synthétiques par Boussif et al en 1995 (Proc. Natl. Acad. Sci. USA, 1995, 92, 7297-7301). On distingue deux types de PEI, selon la structure macromoléculaire; d'une part la PEI à macrostructure linéaire (LPEI, obtenue par hydrolyse de poly(2-alkyl-2-oxazoline)), et d'autre part la PEI à structure branchée (BPEI, obtenue par polymérisation de l'éthylènimine). Ces polymères d'une assez bonne efficacité sont devenus la référence en matière de transfert de gène à l'aide de vecteurs non viraux, bien que cette efficacité soit encore insuffisante pour permettre la mise au point de traitement thérapeutique de maladie héréditaire (Eliyahu et al, Molecules 2005, 10, 34-64). Ces polymères présentent de plus une certaine toxicité, généralement attribuée à leur forte densité de charge positive sur le squelette macromoléculaire. Ces vecteurs ont fait l'objet de nombreuses propositions de modification dans le but soit d'améliorer l'efficacité de transfection, soit de diminuer la toxicité. Ainsi, le brevet US 6,586,524 propose de fixer sur la PEI des branches latérales de poly(éthylène glycol) elles-mêmes fonctionnalisées par un groupement terminal adressable. De même, des poly(ester amine) préparés à partir de polyéther α-ω-diacrylate et de PEI branchée (BPEI) ont montré une efficacité de transfert améliorée avec une toxicité diminuée (Kim et al, Macromolecular Bioscience 2007, 7, 611-619). Cette technique d'amélioration a été largement examinée avec de nombreuses variantes à partir de la BPEI ou avec la LPEI de basse masse molaire (800 Da), notamment en faisant varier la nature du diacrylate, par exemple avec un segment court comme le 1,3-butanediol diacrylate (Forrest et al, Bioconjugate Chemistry 2003, 14, 934-940). Dans ce dernier cas, afin d'obtenir la réticulation ou un produit hautement branché, on dissout la PEI dans du chlorure de méthylène, solvant dans lequel le diacrylate utilisé est également soluble. Dans une publication récente (Arote et al, Biomaterials 2007, 28, 735-744) les auteurs utilisent la poly(éthylènimine) linéaire de basse masse molaire pour réagir avec un diacrylate de polycaprolactone. Dans ce travail on cherche à éviter la réaction des fonctions amines secondaires de la PEI avec les fonctions acrylate en tirant profit du fait que la PEI linéaire possède une fonction amine primaire à chacune de ses extrémités. Dans ce but les auteurs de cet article font la réaction dans le méthanol. Il en est de même dans le travail présenté dans une autre publication où l'on fait réagir de la PEI de basse masse molaire (423 Da) avec du poly(oxyde d'éthylène) diacrylate de masse allant de 250 à 700 Da (Park et al, Journal of Controlled Release 2005, 105, 367-380).

L'utilisation de fonctions imidazolyles pour préparer des vecteurs de transfert polymériques a été décrite dans US 7,163,695 pour un polymère du type polypeptide d'au moins 10 résidus aminoacides. En effet, au moins 10 % de ces aminoacides sont des résidus histidine, mais distribuées de façon non aléatoires. Toutefois dans la classe des polyéthylènimines, seules les BPEI ont également été modifiés par des groupements imidazolyle comme ceux que l'on trouve dans l'histidine. La référence suivante donne des détails sur les diverses tentatives effectuées à ce jour et décrit comment la PEI branchée (BPEI) peut-être modifiée par des fonctions imidazolyle (Swami et al, International Journal of Pharmaceutics 2007, 335, 180-192). Il est à noter que dans cette dernière référence, les auteurs n'utilisent que de la PEI branchée (BPEI)(25 kDa ou 750 kDa), et leur objectif est de réaliser des nanoparticules à l'aide d'une réticulation ultérieure. Cette méthode a été décrite dans cette référence par réaction des groupes amine primaires avec de l'hydrochlorure d'acide 4-imidazoleacétique activé à l'aide de 1-éthyl-3-(3-diméthylaminopropyle) carbodiimide. Il est à remarquer que si toutes ces formulations se sont montrées plus efficaces que la BPEI de départ (la PEI 25 kDa), elles n'atteignent cependant pas l'efficacité des PEI linéaires, bien que ces dernières soient plus toxiques.

Il existe donc dans l'art antérieur de nombreuses façons de modifier les PEI en essayant soit de les rendre moins toxiques, soit d'améliorer leur efficacité de transfection. Toutefois, aucun de ces arts antérieurs n'a permis de trouver un PEI alliant ces deux avantages et en particulier l'efficacité des LPEI avec la faible toxicité des BPEI.

De façon surprenante, les inventeurs ont découverts que la fixation de façon aléatoire sur la polyéthylènimine linéaire de groupements histidyle par l'intermédiaire d'une fonction monoacrylate améliorait sensiblement l'efficacité de transfection tout en diminuant la toxicité cellulaire. Une telle modification permet en effet de garder la structure globalement linéaire de la polyéthylènimine, tout en diminuant sa toxicité et en augmentant la solubilité dans l'eau du polymère ainsi modifié aux pH physiologiques. En d'autres termes, grâce à une telle modification, non seulement on améliore l'efficacité de la LPEI qui est déjà bien meilleure que celle de la BPEI, mais on en diminue largement la toxicité. Par ailleurs cette modification est réalisée en utilisant un procédé simple industriellement.

La présente invention concerne donc un copolymère statistique de polyéthylènimine linéaire comprenant les deux unités monomères de formules I et II suivantes : 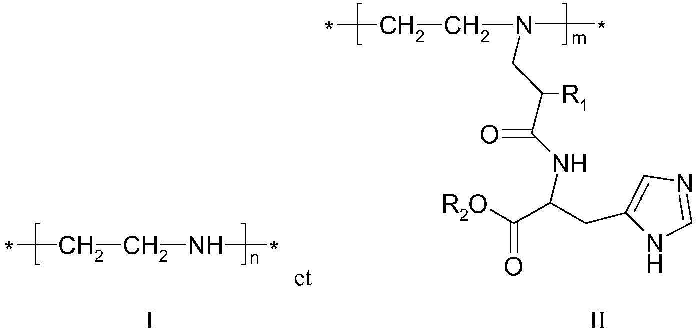 dans lesquels
R₁ représente un atome d'hydrogène ou un groupe alkyle en C₁-C₆,
R₂ représente un atome d'hydrogène, un groupe alkyle en C₁-C₆, aryle ou aralkyle, dans lequel le groupe alkyle est en C₁-C₆,
n est un nombre compris entre 1 et 99 % des monomères totaux et
m est un nombre compris entre 1 et 99 % des monomères totaux.
Avantageusement, ce copolymère a une structure globalement linéaire.

Par le terme « radical alkyle en C₁-C₆ », on entend au sens de la présente invention tout radical alkyle de 1 à 6 atomes de carbones, linéaires ou ramifiés, en particulier, les groupes méthyle, éthyle, n-propyle, iso-propyle, n-butyle, iso-butyle, sec-butyle, t-butyle, n-pentyle, n-hexyle. Avantageusement il s'agit d'un groupe linéaire. De façon avantageuse, il s'agit d'un groupe méthyle.

Par le terme « groupe aryle », on entend au sens de la présente invention un ou plusieurs cycles aromatiques ayant 5 à 8 atomes de carbones, pouvant être accolés. En particulier, les groupes aryles peuvent être des groupes monocycliques ou bicycliques, de préférence phényle, naphtyle, biphényle, tetrahydronaphtyl ou indanyl. Avantageusement il s'agit d'un groupe phényle.

Par le terme « groupe aralkyle », on entend au sens de la présente invention tout groupe aryle tel que défini ci-dessus, lié par l'intermédiaire d'un groupe alkyle tel que défini ci-dessus. En particulier un groupe aralkyle est un groupe benzyle.

Avantageusement R₁ et R₂ identiques ou différents représentent un atome d'hydrogène ou un groupe alkyle en C₁-C₆, de façon avantageuse, un atome d'hydrogène ou un groupe méthyle.

Ainsi le copolymère statistique selon l'invention peut avoir la formule VII suivante : 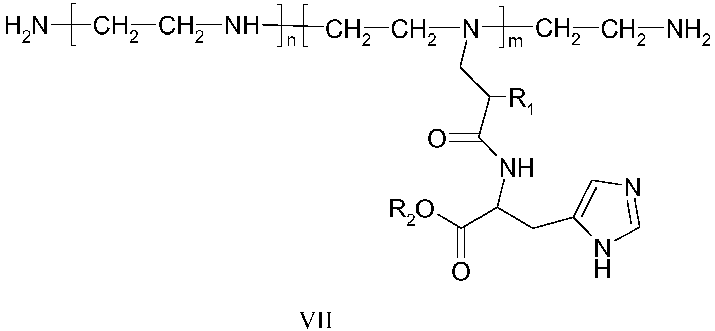 dans laquelle m, n, R₁ et R₂ sont tels que définis précédemment,
ou la formule VIII suivante : 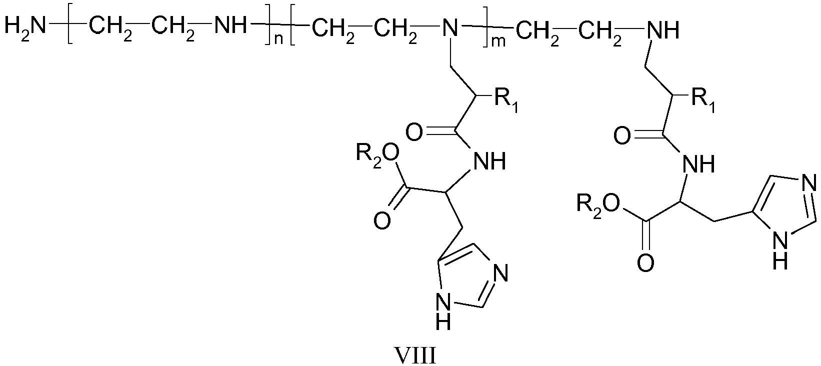 dans laquelle m, n, R₁ et R₂ sont tels que définis précédemment.

L'avantage du copolymère selon l'invention réside dans le fait que l'acide aminé histidine est fixé selon une distribution aléatoire sur une polyéthylènimine linéaire, par l'intermédiaire de sa fonction amine primaire à l'aide d'un bras espaceur court (-CH₂-CH₂-CO-) qui transforme cette fonction amine primaire de l'histidine en fonction amide (-CO-NH-) biocompatible, identique à celle correspondant à la fixation de l'histidine dans les poly(α-amino-acide)s dans les protéines. Ainsi, le copolymère obtenu est un copolymère statistique et n'est pas un copolymère bloc. En effet les copolymères blocs semblent particulièrement désavantageux dans le cadre de la présente invention car chaque bloc ayant des propriétés différentes il y aurait un risque de ségrégation de phase et d'interaction avec une molécule anionique et en particulier l'acide nucléique, différente pour chacune des phases. En outre le copolymère selon l'invention garde globalement la structure linéaire de la LPEI et n'est donc ni branché, ni réticulé.

De plus le copolymère selon l'invention garde le potentiel de charge positive de la PEI puisque l'unité monomère de formule II possède une fonction amine tertiaire toujours susceptible de se charger positivement.

Dans un mode de réalisation avantageux, m est inférieur à 70% des monomères totaux. Avantageusement m est compris entre 1 et 70 % des monomères totaux, plus avantageusement entre 2 et 50 % des monomères totaux, encore plus avantageusement entre 4 et 40% des monomères totaux, de façon avantageuse, entre 5 et 35% des monomères totaux, de façon encore plus avantageuse, entre 10 et 30% des monomères totaux.

Dans un autre mode de réalisation avantageux, n est supérieur à 30 % des monomères totaux, avantageusement n est compris entre 30 et 99 % des monomères totaux, plus avantageusement entre 50 et 98 % des monomères totaux, encore plus avantageusement entre 60 et 96% des monomères totaux, de façon avantageuse entre 65 et 95% des monomères totaux, de façon encore plus avantageuse entre 70 et 90% des monomères totaux.
Avantageusement m + n = 100 %.

De façon avantageuse, le copolymère selon l'invention peut comprendre une unité monomère supplémentaire de formule III suivante : 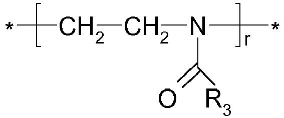 dans laquelle
R₃ représente un groupe alkyle en C₁-C₆, avantageusement un groupe méthyle ou éthyle ;
r est un nombre compris entre 0 et 95 % des monomères totaux, avantageusement entre 0 et 10% des monomères totaux, de façon avantageuse entre 1 et 95% des monomères totaux, de façon encore plus avantageuse entre 1 et 10% des monomères totaux.

Cette unité monomère est également répartie de façon aléatoire dans le copolymère selon l'invention.
Avantageusement m + n + r= 100 %.

Ainsi le copolymère statistique selon l'invention peut avoir la formule IX suivante : 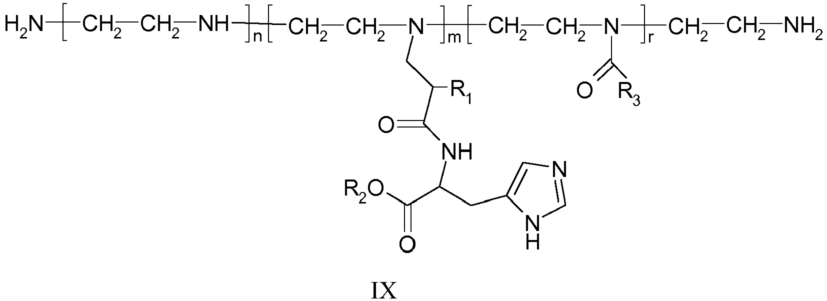 dans laquelle m, n, r, R₁, R₂ et R₃ sont tels que définis précédemment,
ou la formule X suivante : 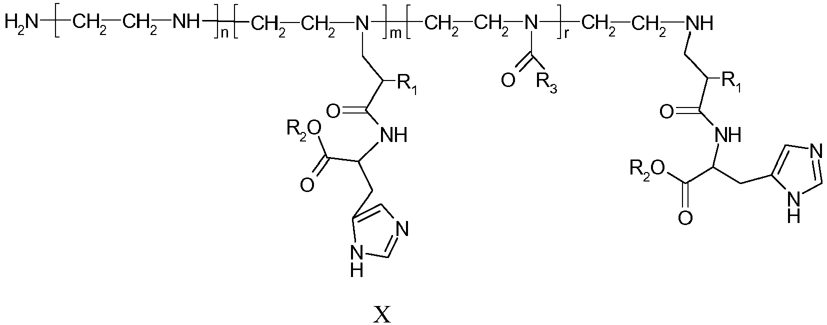 dans laquelle m, n, r, R₁, R₂ et R₃ sont tels que définis précédemment,
ou la formule XI suivante : 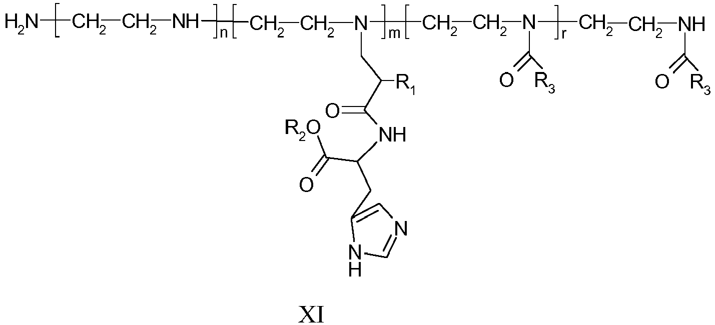 dans laquelle m, n, r, R₁, R₂ et R₃ sont tels que définis précédemment.

Le copolymère selon l'invention peut également comprendre des unités monomères comportant des résidus saccharidiques ou des résidus poly(oxyde d'éthylène) ou des unités peptidiques, avantageusement polylysine.

Les résidus saccharidiques sont avantageusement choisis parmi les résidus de lactose, de tétraglucose, et de mannose.

Les résidus saccharidiques et/ou les résidus poly(oxyde d'éthylène) sont greffés sur l'atome d'azote du motif PEI de formule I, avantageusement par l'intermédiaire d'un groupe C=O.

De façon avantageuse, la proportion de ces résidus est telle que le polyéthylènimine est toujours globalement de structure linéaire. Avantageusement chacun de ces résidus est présent dans le copolymère en une proportion inférieure à 70% des monomères totaux, de façon avantageuse, en une proportion inférieure à 50% des monomères totaux et de façon encore plus avantageuse en une proportion inférieure à 30% des monomères totaux, encore plus avantageusement en une proportion inférieure à 10% des monomères totaux. De façon avantageuse, chacune de ces unités est disposée aléatoirement dans le copolymère.

La présente invention concerne en outre un procédé de préparation d'un copolymère selon l'invention, caractérisé en ce qu'il comprend l'étape de mise en contact d'une polyéthylènimine linéaire solubilisée dans de l'eau à un pH compris entre 6,5 et 7,5 avec le composé de formule IV suivante 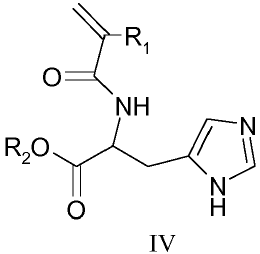 dans laquelle R₁ et R₂ sont tels que définis précédemment, avantageusement à la température de reflux de l'eau.

Le procédé selon l'invention peut donc être mis en oeuvre en phase aqueuse ce qui constitue une grande simplification d'une part et un avantage puisqu'une poly(éthylènimine) linéaire de masse molaire élevée (par exemple la PEI 22 kDa) peut être ainsi modifiée.

Ce procédé consiste donc en la modification aléatoire d'une polyéthylènimine linéaire par réaction avec un composé de formule IV.

Cette réaction permet donc de modifier la fonction amine secondaire de la PEI en fonction amine tertiaire. Cette réaction d'addition conduit à une transformation partielle et aléatoire des motifs éthylènimine -CH₂-CH₂-NH- en motifs monomères substitués sur l'azote par des groupements : 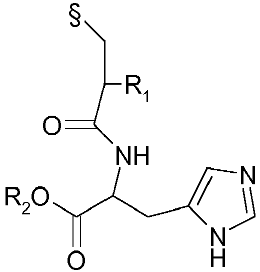 dans lesquels § indique l'endroit où se greffe la molécule sur l'azote et R₁ et R₂ sont tels que définis précédemment.

En outre, le composé de formule IV étant un monoacrylate, son utilisation permet de garder au squelette macromoléculaire de la PEI ainsi modifié sa structure globalement linéaire et d'éviter la formation de polymère branché ou réticulé.

Les modifications de la poly(éthylènimine) par le procédé selon l'invention permettent de garder le potentiel de charge positive de la PEI puisque cette modification transforme une fonction amine secondaire en fonction amine tertiaire toujours susceptible de se charger positivement.

Le taux de modification de la PEI et donc la valeur de m dans le copolymère selon l'invention dépend de la durée de l'étape de mise en contact du composé de formule IV avec la LPEI.

Avantageusement, la durée de cette étape est au maximum de 5 jours, de façon avantageuse, au maximum de 4 jours, de façon plus avantageuse au maximum de 3 jours, plus avantageusement au minimum de 12 heures, encore plus avantageusement au minimum de 24 heures.

Dans un mode de réalisation avantageux, la polyéthylènimine linéaire avant modification a une masse molaire moyenne en nombre comprise entre 400 Da et 1 000 000 de Da, avantageusement entre 4000 et 40 000 Da, encore plus avantageusement entre 10 000 et 30 000 Da.

De façon avantageuse, la LPEI qui va réagir avec le composé de formule IV est un copolymère statistique comprenant les deux unités monomères de formules I et III telles que définies précédemment.

Ainsi ce copolymère statistique peut avoir la formule XII suivante : 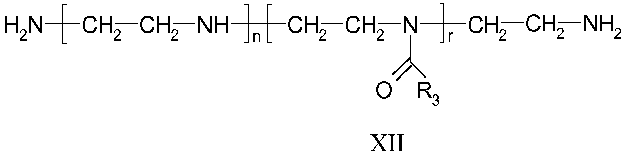 dans laquelle n, r et R₃ sont tels que définis précédemment
ou la formule XIII suivante : 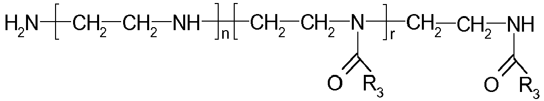 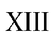 dans laquelle n, r et R₃ sont tels que définis précédemment.

Ce copolymère a une structure globalement linéaire.

La LPEI qui va réagir avec le composé de formule IV peut également comprendre des unités monomères comportant des résidus saccharidiques ou des résidus poly(oxyde d'éthylène) ou des unités peptidiques, avantageusement polylysine. Les résidus saccharidiques sont avantageusement choisis parmi les résidus de lactose, de tétraglucose, et de mannose.

Les résidus saccharidiques et/ou les résidus poly(oxyde d'éthylène) sont greffés sur l'atome d'azote du motif PEI de formule I, avantageusement par l'intermédiaire d'un groupe C=O.

De façon avantageuse, la proportion de ces résidus est telle que la polyéthylènimine est toujours globalement de structure linéaire. Avantageusement chacun de ces résidus est présent dans le copolymère en une proportion inférieure à 70%, de façon avantageuse, en une proportion inférieure à 50% et de façon encore plus avantageuse en une proportion inférieure à 30%, encore plus avantageusement en une proportion inférieure à 10%.

De façon avantageuse, chacun de ces résidus est réparti aléatoirement dans la LPEI.

La réaction entre la LPEI et le composé de formule générale IV ne modifie pas les autres éventuelles unités monomères présentes dans la LPEI.

Avantageusement la LPEI qui va réagir avec le composé de formule IV est obtenue par hydrolyse acide de poly(2-R₃-2-oxazoline) avec R₃ tel que défini précédemment. Les procédés de préparation de la LPEI sont bien connus de l'homme du métier, que la LPEI contienne ou non les unités monomères de formule III et/ou les unités monomères comportant des résidus saccharidiques ou des résidus poly(oxyde d'éthylène) et/ou des unités peptidiques, avantageusement polylysine. En particulier l'unité monomère de formule III telle que définie précédemment peut provenir par exemple d'une hydrolyse incomplète de la poly(2-R₃-2-oxazoline) de départ.

Le composé de formule générale IV peut être obtenu par réaction du composé de formule générale V suivante 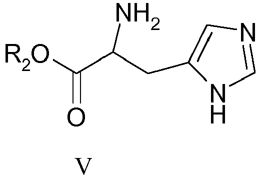 dans lequel R₂ est tel que défini précédemment avec un composé de formule générale VI suivante 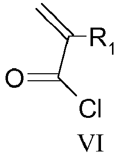 dans laquelle R₁ est tel que défini précédemment, avantageusement en milieu basique, de façon avantageuse, en présence de soude, en particulier en phase aqueuse. Ce procédé a en particulier été décrit dans l'article de Bentolila et al (J. Med. Chem. 2000, 43, 2591-2600).

La présente invention concerne en outre une composition pharmaceutique comprenant un copolymère selon la présente invention en tant que vecteur pharmaceutiquement acceptable, une substance active et éventuellement un autre excipient pharmaceutiquement acceptable.

En effet, le copolymère selon l'invention peut être utilisé pour acheminer une substance active vers l'endroit exact où elle doit entrer en action dans un organe, un tissu ou une cellule.

Par le terme de « substance active », on entend au sens de la présente invention tout principe actif pharmaceutique (antalgiques, antipyrétiques, aspirine et dérivés, antibiotiques, anti-inflammatoires, antiulcéreux, antihypertenseurs, neuroleptiques, antidépresseurs, oligonucléotides, peptides, protéines par exemple) ; cosmétique (anti UV, autobronzant par exemple), nutraceutique (vitamines par exemple) ; alimentaire ; agrochimique ou vétérinaire. Avantageusement cette substance active est chargée négativement. Il s'agit donc dans ce cas d'une molécule anionique.

Ces compositions peuvent être formulées pour l'administration aux mammifères, y compris l'homme. La posologie varie selon le traitement et selon l'affection en cause. Ces compositions sont réalisées de façon à pouvoir être administrées par la voie orale, sublinguale, sous-cutanée, intramusculaire, intraveineuse, transdermique, locale ou rectale. Les formes unitaires d'administrations appropriées comprennent les formes par voie orale telles que les comprimés, les gélules, les poudres, les granules et les solutions ou suspensions orales, les formes d'administration sublinguale et buccale, les formes d'administration sous-cutanée, intramusculaire, intraveineuse, intranasale ou intraoculaire et les formes d'administration rectale.

Ainsi la composition pharmaceutique selon la présente invention peut comprendre un autre excipient pharmaceutiquement acceptable tel que la gélatine, l'amidon, le lactose, le stéarate de magnésium, le talc, la gomme arabique ou analogues, un édulcorant, un antiseptique, ainsi qu'un agent donnant du goût, un colorant approprié, des agents de dispersion ou des agents mouillants, des agents de mise en suspension ou un diluant. On peut enrober les comprimés de la composition selon l'invention avec du saccharose ou d'autres matières appropriées ou encore on peut les traiter de telle sorte qu'ils aient une activité prolongée ou retardée et qu'ils libèrent d'une façon continue une quantité prédéterminée du principe actif et du copolymère selon l'invention.

Pour une administration rectale, on recourt à des suppositoires qui sont préparés avec des liants fondant à la température rectale, par exemple du beurre de cacao ou des polyéthylèneglycols.

Pour une administration parentérale, intranasale ou intraoculaire, on utilise des suspensions aqueuses, des solutions salines isotoniques ou des solutions stériles et injectables qui contiennent des agents de dispersion et/ou des agents mouillants pharmacologiquement compatibles.

La présente invention concerne en outre l'utilisation du copolymère selon la présente invention en tant que vecteur pharmaceutiquement acceptable d'une substance active dans une composition pharmaceutique.

La présente invention concerne de plus un complexe comprenant
a) le copolymère selon la présente invention,
b) au moins une molécule anionique.

Dans un tel complexe, la liaison entre le composé a) et le composé b) est de nature électrostatique. En effet, dans le cadre des complexes selon l'invention, le copolymère selon l'invention se trouve sous une forme cationique Avantageusement la molécule anionique est un acide nucléique.

Dans le complexe selon la présente invention, l'acide nucléique peut être aussi bien un acide désoxyribonucléique (ADN) qu'un acide ribonucléique (ARN) ou des séquences hybrides ADN/ARN. Il peut s'agir de séquences d'origine naturelle ou artificielle, et notamment d'ADN génomique, d'ADN complémentaire (ADNc), d'ARN messager (ARNm), d'ARN de transfert (ARNt), d'ARN ribosomique (ARNr), ou de séquences synthétiques ou semi-synthétiques. En outre, l'acide nucléique peut avoir une taille très variable allant de l'oligonucléotide au chromosome. Ces acides nucléiques peuvent être d'origine humaine, animale, végétale, bactérienne, virale, etc. Ils peuvent être obtenus par toute technique connue de l'homme du métier, et notamment par criblage de banques, par synthèse chimique, ou encore par des méthodes mixtes incluant la modification chimique ou enzymatique de séquences obtenues par criblage de banques. Ils peuvent également être obtenus par une modification chimique au niveau de leurs parties sucres, de leurs parties nucléobases ou de leur squelette internucléotidique. Parmi les modifications avantageuses dans les parties sucres, on peut notamment citer les modifications intervenant en position 2' du ribose, telles que les modifications 2'-deoxy, 2'-fluoro, 2'-amino, 2'-thio, ou 2'-O-alkyl, en particulier 2'-O-méthyle, au lieu du groupement 2'-OH normal sur les ribonucléotides, ou encore la présence d'un pont méthylène entre les positions 2' et 4' du ribose (LNA). Concernant les nucléobases, il est possible d'utiliser des bases modifiées, telles que notamment la 5-bromo-uridine, la 5-iodo-uridine, la N³-méthyl-uridine, une 2,6-diaminopurine (DAP), la 5-méthyl-2'-deoxyCytidine, la 5-(1-propynyl)-2'-deoxy-Uridine (pdU), la 5-(1-propynyl)-2'-deoxyCytidine (pdC), ou des bases conjuguées avec du cholestérol. Enfin, des modifications avantageuses du squelette internucléotidique comprennent le remplacement de groupements phosphodiesters de ce squelette par des groupements phosphorothioate, méthylphosphonate, phosphorodiamidate, ou l'utilisation d'un squelette composé d'unités de N-(2-aminoethyl)-glycine liées par des liaisons peptidiques (PNA, Peptide Nucleic Acid). Les différentes modifications (base, sucre, squelette) peuvent bien entendu être combinées pour donner des acides nucléiques modifiés de type morpholino (bases fixées sur un cycle morpholine et liées par des groupes phosphorodiamidate) ou PNA (bases fixées sur des unités de N-(2-aminoethyl)-glycine liées par des liaisons peptidiques).

Ils peuvent par ailleurs être incorporés dans des vecteurs, tels que des vecteurs plasmidiques.

Avantageusement l'acide nucléique est choisi dans le groupe constitué par de l'ARN, de l'ADN complémentaire (ADNc), de l'ADN génomique, de l'ADN plasmidique, de l'ADN antisens, de l'ARN messager, de l'ARN antisens, de l'ARN interférant, des ribozymes, de l'ARN de transfert, de l'ARN ribosomique, ou de l'ADN codant ces types d'ARN.

Concernant plus particulièrement les acides désoxyribonucléiques, ils peuvent être simple ou double brin. Ces acides nucléiques peuvent comprendre une séquence de gènes choisis parmi a) des gènes marqueurs, b) des gènes à visée thérapeutique et c) des gènes à visée vaccinale, et les éléments permettant son expression.

Au sens de l'invention, on entend par « gène à visée thérapeutique » notamment tout gène codant pour un produit protéique ayant un effet thérapeutique. Le produit protéique ainsi codé peut être une protéine, un peptide, etc. Ce produit protéique peut être homologue vis-à-vis de la cellule cible (c'est-à-dire un produit qui est normalement exprimé dans la cellule cible lorsque celle-ci ne présente aucune pathologie). Dans ce cas, l'expression d'une protéine permet par exemple de pallier à une expression insuffisante dans la cellule ou à l'expression d'une protéine inactive ou faiblement active en raison d'une modification, ou encore de surexprimer ladite protéine. Le gène thérapeutique peut aussi coder pour un mutant d'une protéine cellulaire, ayant une stabilité accrue, une activité modifiée, etc. Le produit protéique peut également être hétérologue vis-à-vis de la cellule cible. Dans ce cas, une protéine exprimée peut par exemple compléter ou apporter une activité déficiente dans la cellule, lui permettant de lutter contre une pathologie, ou stimuler une réponse immunitaire. Le gène thérapeutique peut également coder pour une protéine sécrétée dans l'organisme.

Le gène thérapeutique peut également être un gène ou une séquence antisens, dont l'expression dans la cellule cible permet de contrôler l'expression de gènes ou la transcription d'ARN cellulaires. De telles séquences peuvent par exemple, être transcrites dans la cellule cible en ARN complémentaire d'ARNm cellulaires et bloquer ainsi leur traduction en protéine. Il peut s'agir aussi d'oligonucléotides synthétiques, éventuellement modifiés. Les antisens comprennent également les séquences codant pour des ribozymes, qui sont capables de détruire sélectivement des ARN cibles. Le gène thérapeutique peut également être un gène codant pour un siRNA ou un shRNA.

Avantageusement les gènes à visée thérapeutique sont choisis parmi les gènes codant :
- la dystrophine et minidystrophine (myopathies) ;
- la protéine CFTR cystic fibrosis transmembrane conductance regulator associée à la mucoviscidose ;
- l'alpha1-antitrypsine ;
- l'Erythropoïétine (EPO);
- les cytokines telles que les interleukines et le TNF facteur de nécrose des tumeurs ;
- les facteurs de croissance tels que le TGFbéta et le PDGF ;
- les enzymes lysosomiques telles que la béta-glucosidase ;
- l'adénosine désaminase (ADA) ;
- des ARN sens et antisens ;
- des ARN interférants ;
- des ribozymes ;
- les récepteurs des lipoprotéines de faible densité, déficient dans le cas d'hypercholestérolémie ;
- les facteurs de coagulation tels que les facteurs VII, VIII et IX ;
- la phénylalanine-hydroxylase ;
- la tyrosine hydroxylase ;
- la thymidine kinase du virus Herpes simplex ;
- les protéines du complexe majeur d'histocompatibilité, avantageusement les HLA-B7 ;
- la cytosine désaminase ;

Comme indiqué plus haut, l'acide nucléique peut également comporter un ou plusieurs gènes à visée vaccinale capable de générer chez l'homme ou l'animal une réponse immunitaire. Dans ce mode particulier de mise en oeuvre, l'invention permet donc la réalisation soit de vaccins soit de traitements immunothérapeutiques appliqués à l'homme ou à l'animal, notamment contre des microorganismes, des parasites, des bactéries, des virus ou des cancers. Il peut s'agir notamment de peptides antigéniques spécifiques du virus d'Epstein Barr, du virus HIV, du virus de l'hépatite B, du virus de la pseudo-rage, ou encore spécifiques de tumeurs.

Ainsi, avantageusement les gènes à visée vaccinale sont choisis parmi les gènes codants pour:
- des antigènes viraux, avantageusement la nucléoprotéine du virus de la grippe ou la protéine NEF ou GAG du virus VIH ;
- des antigènes associés aux tumeurs, avantageusement l'antigène MART1 des cancers à mélanome, les mucines ou l'antigène PSA des cancers de la prostate ;
- des antigènes bactériens ;
- des antigènes parasitaires.

Dans un autre mode de réalisation avantageux, le gène marqueur est choisi parmi les gènes de :
- la luciférase de la luciole ;
- la protéine verte de méduse *Aequora Victoria ;*
- la béta-galactosidase d'E. Coli ;
- la chloramphénicol acétyle transférase ;
- résistance à un antibiotique, avantageusement de résistance à la néomycine ou à l'hygromycine.

Pour obtenir un effet optimum des complexes de l'invention, les proportions respectives du polymère et de l'acide nucléique sont de préférence déterminées de sorte que le rapport massique (µg/µg) entre le polymère et l'acide nucléique soit compris entre 1/3 et 1/4.

Ce rapport peut bien entendu être adapté par l'homme du métier en fonction du copolymère utilisé, de la présence d'un adjuvant (voir ci-après), de l'acide nucléique, de la cellule cible et du mode d'administration utilisé.

Les complexes selon l'invention peuvent être préparés en mélangeant une solution de l'acide nucléique concerné et une solution du copolymère selon l'invention. Avantageusement ces solutions sont préparés à partir de sérum physiologique ou d'un tampon, tel que le tampon hepes, ou d'un milieu cytocompatible.

La présente invention concerne en outre une composition pharmaceutique comprenant un complexe selon l'invention et éventuellement un excipient pharmaceutiquement acceptable.

La composition pharmaceutique selon l'invention peut être utilisée telle quelle ou en association avec d'autres composés. Ainsi, dans un mode particulier de mise en oeuvre, la composition selon la présente invention comprend en plus un adjuvant capable de s'associer au complexe copolymère/acide nucléique selon l'invention et d'améliorer le pouvoir transfectant. En effet, le pouvoir transfectant des compositions de l'invention peut être encore amélioré en présence de certains adjuvants (lipides, protéines, lipopolyamines, polymères synthétiques par exemple), capables de s'associer au complexe copolymère/acide nucléique selon l'invention.

Avantageusement, les adjuvants utilisés dans les compositions selon l'invention sont des lipides cationiques (comportant une ou plusieurs charges cationiques dans leur partie polaire) ou des lipides neutres.

Concernant les lipides cationiques, il peut s'agir plus particulièrement de lipopolyamine. D'autres adjuvants particulièrement avantageux pour la réalisation des compositions de l'invention sont représentés par les lipides neutres. L'utilisation de lipides neutres est particulièrement avantageuse lorsque le rapport de charges R (amines/phosphates) est faible. De manière particulièrement avantageuse, on utilise des lipides naturels ou synthétiques, zwitterioniques ou dépourvus de charge ionique dans les conditions physiologiques. Ces différents lipides peuvent être obtenus soit par synthèse, soit par extraction à partir d'organes (exemple : le cerveau) ou d'oeufs, par des techniques classiques bien connues de l'homme du métier.

Dans un mode de réalisation particulièrement avantageux, les compositions de la présente invention comprennent un élément de ciblage permettant d'orienter le transfert de l'acide nucléique. Cet élément de ciblage peut être un élément de ciblage extracellulaire, permettant d'orienter le transfert de l'acide nucléique vers certains types cellulaires ou certains tissus souhaités (cellules tumorales, cellules hépatiques, cellules hématopoïétiques, etc). Il peut également s'agir d'un élément de ciblage intracellulaire, permettant d'orienter le transfert de l'acide nucléique vers certains compartiments cellulaires privilégiés (mitochondries, noyau, etc). Parmi les éléments de ciblage utilisables dans le cadre de l'invention, on peut citer les sucres, les peptides, les oligonucléotides, les lipides ou les protéines. Avantageusement, il s'agit de sucres, de peptides ou de protéines tels que des anticorps ou fragments d'anticorps, des ligands de récepteurs cellulaires ou des fragments de ceux-ci, des récepteurs ou fragments de récepteurs, etc. En particulier, il peut s'agir de ligands de récepteurs de facteurs de croissance, de récepteurs de cytokines, de récepteurs de lectines cellulaires ou de récepteurs de protéines d'adhésion. On peut également citer le récepteur de la transferrine, des HDL et des LDL. L'élément de ciblage peut également être un sucre permettant de cibler les récepteurs asialoglycoprotéiques, ou encore un fragment Fab d'anticorps permettant de cibler le récepteur du fragment Fc des immunoglobulines.

Les compositions selon l'invention peuvent être formulées en vue d'administrations par voie topique, cutanée, orale, rectale, vaginale, parentérale, intranasale, intraveineuse, intramusculaire, sous-cutanée, intraoculaire, transdermique, etc. De préférence, les compositions pharmaceutiques de l'invention contiennent un véhicule pharmaceutiquement acceptable pour une formulation injectable, notamment pour une injection directe au niveau de l'organe désiré, ou pour une administration par voie topique (sur peau et/ou muqueuse). Il peut s'agir en particulier de solutions stériles, isotoniques, ou de compositions sèches, notamment lyophilisées, qui, par addition selon le cas d'eau stérilisée ou de sérum physiologique, permettent la constitution de solutés injectables. Les doses d'acide nucléique utilisées pour l'injection ainsi que le nombre d'administrations peuvent être adaptées en fonction de différents paramètres, et notamment en fonction du mode d'administration utilisé, de la pathologie concernée, du gène à exprimer, ou encore de la durée du traitement recherchée.

Comme indiqué ci-avant, les complexes et compositions selon l'invention peuvent être utilisées pour le transfert d'acides nucléiques dans les cellules *in vivo, in vitro* ou *ex vivo.* En particulier les copolymères selon l'invention peuvent être utilisés pour transférer de manière très efficace des acides nucléiques dans de nombreux types cellulaires, et en particulier dans certains types cellulaires habituellement difficiles à transfecter.

Ainsi il est décrit un procédé pour transférer *in vitro* ou *ex vivo* un acide nucléique dans des cellules, ledit procédé comprenant la mise en contact desdites cellules avec au moins un complexe selon la présente invention ou une composition pharmaceutique selon l'invention.

Les cellules peuvent être d'origine procaryote ou eucaryote, en particulier animale, végétale ou humaine.

Avantageusement, les cellules sont des cellules de mammifères, de façon avantageuse choisies parmi :
- des cellules souches hématopoïétiques ;
- des cellules du système immunitaire, telles que des cellules dendritiques, des macrophages et des lymphocytes ;
- des cellules du foie ;
- des cellules des muscles squelettiques ;
- des cellules de la peau, telles que des fibroblastes, des kératinocytes, des cellules dendritiques et des mélanocytes ;
- des cellules des vaisseaux et microvaisseaux sanguins, telles que des cellules endothéliales et des cellules musculaires lisses ;
- des cellules épithéliales des voies aériennes ;
- des cellules du système nerveux central ;
- des cellules cancéreuses ;
- des cellules du tissu conjonctif, telles que des ténocytes.

Avantageusement, la méthode de transfection *in vitro* ou *ex vivo,* se caractérise en ce que l'on met en présence un complexe ou une composition pharmaceutique selon l'invention dans un milieu contenant des cellules à transfecter, dans des conditions telles qu'il y a:
- passage du complexe à partir du milieu dans le cytoplasme des cellules,
- relargage de l'acide nucléique impliqué dans le susdit complexe dans le cytosol et/ou le noyau des cellules,
- transcription et expression de l'acide nucléique dans les cellules transfectées,
- expression de la protéine correspondant au gène transfecté.

La présente invention concerne de plus l'utilisation d'un copolymère selon l'invention pour la préparation d'une composition pharmaceutique destinée à la délivrance intracellulaire de molécules anioniques, avantageusement d'acide nucléique.

Elle concerne en outre un complexe selon l'invention pour son utilisation en tant que médicament destiné à la délivrance intracellulaire d'acide nucléique, avantageusement destiné au traitement de tumeurs, plus avantageusement de léiomyome utérin ou de tumeur maligne telle que le carcinome des ovaires, du sein ou de l'endomètre, pour la préparation d'un vaccin ou au traitement ou à la prévention de déficience métabolique congénitale ou acquise ou de maladies associées avec une expression génétique déficiente, plus avantageusement au traitement de maladies liées aux troubles d'un gène unique telles que les syndromes de déficit immunitaire combiné sévère, la mucoviscidose, l'hémophilie, la drépanocytose, la béta-thalassémie et la dystrophie musculaire.

L'invention sera mieux comprise à la lecture des exemples et des figures qui suivent.

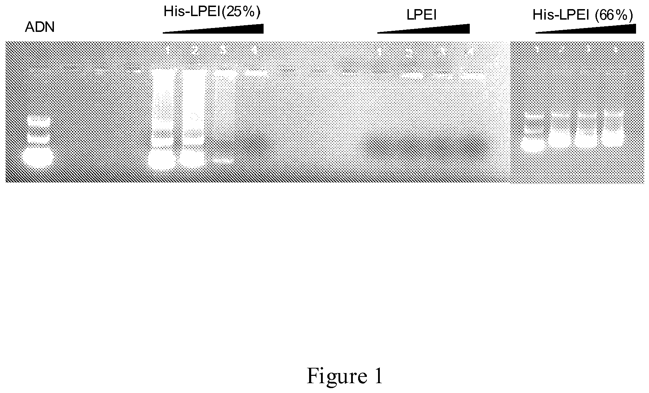

La figure 1 représente la migration de l'ADN plasmidique en électrophorèse dans un gel d'agarose en présence de bromure d'éthydium. La piste ADN correspond à la migration de 1 µg d'ADN en absence de polymère. Pour le copolymère selon l'invention avec m= 25% et R₁ et R₂₌H (His-LPEI (25%)), un LPEI non modifié (LPEI) et le copolymère selon l'invention avec m= 66% et R₁ et R₂₌H (His-LPEI (66%)), les pistes 1, 2, 3 et 4 correspondent à la migration de 1µg d'ADN en présence de 1, 2, 3 et 4 µg de polymère ou copolymère. L'ADN est révélé sous lumière UV..

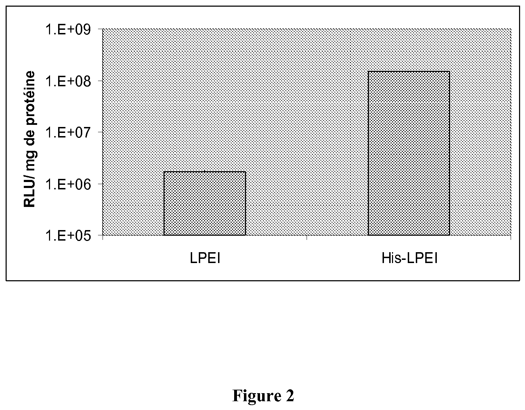

La figure 2 représente la mesure de l'activité luciférase (RLU) 48 h après la transfection de cellules HeLa avec 2,5 µg de plasmide codant la luciférase complexé à la LPEI non modifiée (LPEI) ou au copolymère selon l'invention avec m= 25% et R₁ et R₂=H (His-LPEI).

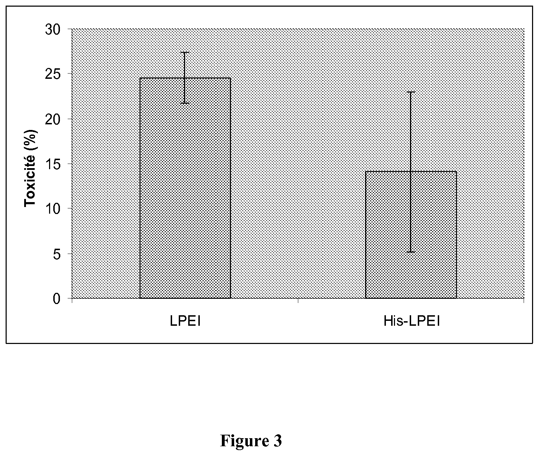

La figure 3 représente la mesure de la toxicité induite pas la transfection des cellules HeLa avec 2,5 µg de plasmide codant la luciférase complexés à la LPEI non modifiée (LPEI) ou au copolymère selon l'invention avec m= 25% et R₁ et R₂₌H (His-LPEI). La viabilité cellulaire est mesurée 48 h après la transfection par un dosage colorimétrique utilisant le MTT.

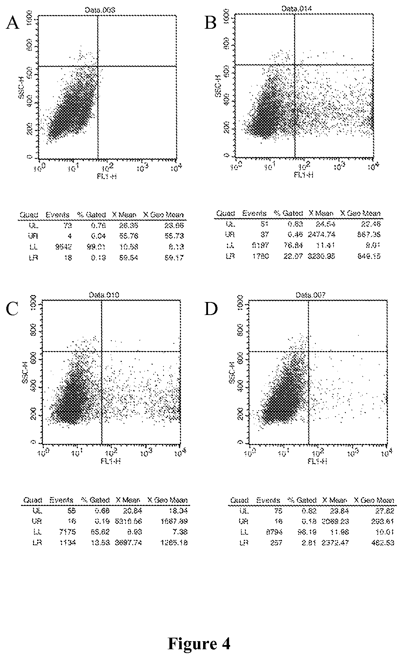

La figure 4 représente l'évaluation du nombre de cellules HeLa transfectées avec 2,5 µg de plasmide codant la protéine verte de la méduse (pCMV-EGFP) complexés à la LPEI non modifiée (D) ou au copolymère selon l'invention avec R₁ et R₂=H et m= 25% (B) ou m = 4% (C) comparés avec les cellules non transfectées (A). Le nombre de cellules exprimant la EGFP est déterminé après mesure de la fluorescence des cellules (FL1-H) par cytométrie en flux 48 h après la transfection.

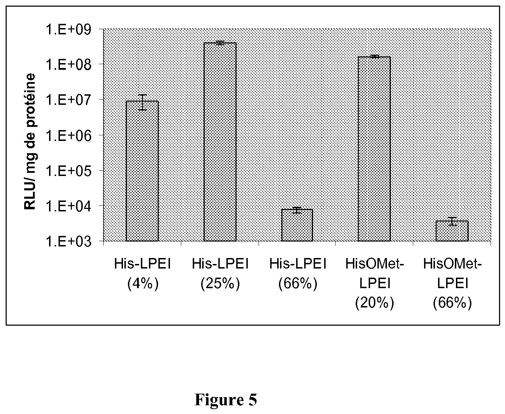

La figure 5 représente la mesure de l'activité luciférase (RLU) 48 h après la transfection de cellules HeLa avec 2,5 µg de plasmide codant la luciférase complexés au copolymère selon l'invention avec R₁ et R₂=H et m= 4%, 25% ou 66% ou au copolymère selon l'invention avec R₁=H et R₂=méthyle et m= 20% ou 66%.

Les exemples suivants illustrent l'invention sans en limiter la portée.

Les copolymères de polyéthylènimines selon l'invention sont testés pour leurs propriétés de transfection de diverses lignées cellulaires à l'aide, par exemple, de plasmide codant pour la luciférase.

### Exemple 1 : copolymère selon l'invention : R₁=R₂=H, m=25% et complexe avec l'ADN d'un gène rapporteur codant la luciférase

### Préparation du copolymère selon l'invention :

On prépare un copolymère de LPEI selon l'invention avec R₁ = R₂ = H et m= 25 % de la façon suivante:
On commence par la préparation de la poly(éthylènimine) linéaire LPEI à partir de la poly(2-méthyl-2-oxazoline) par hydrolyse acide :
10 g de poly(2-méthyl-2-oxazoline) de masse molaire 50.000 Da (Aldrich) sont dissous dans 115 mL d'eau distillée, auxquels on ajoute 75 mL d'acide chlorhydrique à 37 % (Aldrich). On chauffe à reflux pendant 24 heures. Après refroidissement on ajoute des pastilles de soude pour amener le milieu réactionnel à pH = 14. La LPEI précipite. On lave le précipité à l'eau distillée jusqu'à l'obtention d'un lavage neutre. On lave ensuite le précipité par de l'acétone et la LPEI ainsi obtenue est séchée sous vide. Le rendement est de 98 % et le produit est vérifié par résonance magnétique nucléaire.

La synthèse de l'acrylamide N-2-(3(3H-imidazol-4-yl)propanoique acide)) (produit de formule IV, R₁ et R₂ =H) s'effectue de la façon suivante : 3,1g d'histidine (Aldrich) sont dissous dans 100mL d'eau distillée contenant 3g de soude. 1,8g de chlorure d'acryloyle (Aldrich) sont ajoutés goutte à goutte sous agitation à température ordinaire. On récupère le produit recherché par concentration du milieu réactionnel, précipitation dans l'acétone et filtration. Le produit est ensuite séché sous vide.

La modification de la LPEI s'effectue de la façon suivante : 3,7 g de LPEI préparée comme ci-dessus, 3 g d' acrylamide N-2-(3(3H-imidazol-4-yl)propanoique acide) sont dissous dans 40 mL d'eau distillée. On chauffe à reflux pendant 2 jours. Après refroidissement à température ordinaire, on évapore à sec et on reprend avec 20 mL de méthanol. La partie non soluble dans le méthanol est le produit cherché. On termine par un séchage sous vide. Ce mode opératoire permet d'obtenir le copolymère selon l'invention avec 25 % de modification des motifs éthylènimine (m=25%).

L'efficacité de transfection du copolymère selon l'invention obtenu ci-dessus a été testée in vitro et mesurée à l'aide d'un gène rapporteur codant la luciférase sous le contrôle du promoteur CMV. Elle a été comparée avec l'efficacité de transfection d'un LPEI non modifié.

### Préparation de complexes selon l'invention et de complexe LPEI non modifié/ ADN:

Pour déterminer la formation optimale des complexes ADN/copolymère selon l'invention et ADN/LPEI non modifié, différents mélanges sont préparés selon la procédure suivante et analysés par électrophorèse en gel d'agarose à 0,6 % : 30 µl d'une solution de polymère à raison de 1 mg/mL sont dilués dans 60 µL de tampon Hepes 10 mM, pH 7,4. Cette solution de polymère est ensuite transférée dans 140 µL de tampon hepes 10mM, pH 7,4 contenant 7,5 µg de plasmide codant pour la luciférase. Le mélange est agité fortement pendant 4s puis laissé au repos à 20°C pendant 30 min. 20µL du mélange sont analysés par électrophorèse en gel d'agarose à 0,6 % contenant du bromure d'éthydium permettant la révélation de l'ADN par irradiation UV. Les mélanges permettant une totale non-migration de l'ADN sont considérés comme étant ceux qui contiennent de l'ADN totalement condensé avec un polymère (Figure 1).Ces complexes sont ceux qui seront utilisés pour la transfection des cellules, c'est à dire pour les complexes ADN/LPEI non modifié dans un rapport massique 1/2 (µg :µg), ou 1/3 (µg :µg) et 1/4 (µg :µg) pour les complexes ADN/copolymère selon l'invention. Comme l'indique le gel d'agarose, lorsque la LPEI est substituée à raison de 66 % avec de l'histidine (copolymère selon l'invention avec R₁=R₂=H et m=66%), les interactions du polymère avec l'ADN sont faibles et il y a absence de condensation forte de l'ADN. La taille et la charge globale (potentiel zeta) des complexes sont mesurées à l'aide du zetasizer 3000 (Malvern Instruments). Les complexes ADN/LPEI non modifié ont une taille de 78 nm et un potentiel zeta de 13 mV, la taille des complexes ADN/LPEI selon l'invention avec R₁=R₂=H et m= 25% est de 250 nm et le potentiel zeta est de 8 mV.

### Essais de transfection in vitro:

Les cellules HeLa ont été ensemencées dans une plaque à 24 puits à la densité initiale de 2 x 10⁵ cellules par puits. Elles ont subi une incubation pendant 18-20 heures pour atteindre une confluence de 80%. Les solutions de complexes ADN/polymère décrits ci-dessus sont ajustées à 1,5 mL avec du milieu de culture cellulaire contenant 10 % de sérum de veau fétal. Le milieu des cellules est alors remplacé par 0,5 mL du mélange contenant le complexe LPEI/ADN codant pour la luciférase (2,5 µg/puits) ou le complexe copolymère selon l'invention (avec R₁=R₂=H et m= 25 %)/ADN codant pour la luciférase (2,5 µg/puits). Une incubation de 4 heures est ensuite réalisée à 37°C dans une étuve avec 95 % d'air et 5 % de CO₂. Le milieu est ensuite remplacé par un milieu contenant 10 % de sérum de veau fétal et maintenu pendant 48 heures dans un incubateur à 37°C. Les essais d'activité luciférase sont réalisés selon le protocole décrits dans Pichon et al. (J. Gene Med. 2002, 4 , 548-559), et l'intensité de lumière émise a été ensuite normalisée à la quantité de protéine produite après un dosage BCA selon le mode opératoire décrit dans la même référence.

### Les résultats sont les suivants :

La LPEI non modifiée donne une activité luciférase de 1,7.10⁶ RLU/mg de protéine tandis que le copolymère selon l'invention avec R₁=R₂=H et m = 25 % donne une activité luciférase de 1,5.10⁸ RLU/mg de protéine (Figure 2).

Ainsi, non seulement l'efficacité de transfection est augmentée, mais la toxicité est largement diminuée : en utilisant le dosage MTT, la toxicité mesurée est de 26 % pour la LPEI non modifiée, elle n'est que de 14 % pour le copolymère selon l'invention avec R₁=R₂=H et m = 25 % (Figure 3).

### Exemple 2 : copolymère selon l'invention : R₁=R₂=H, m=25% et complexe avec l'ADN d'un plasmide codant pour la protéine verte fluorescente de méduse

On fait les mêmes essais de transfection que dans l'exemple 1 à partir du copolymère obtenu à l'exemple 1 mais on utilise ici un plasmide codant pour la protéine verte fluorescente de méduse (EGFP). La mesure de la fluorescence des cellules en cytométrie de flux permet d'atteindre le nombre de cellules transfectées ainsi que l'intensité de l'expression du gène dans les cellules et la viabilité des cellules en présence de l'iodure de propidium. Les résultats montrés dans la figure 4 indiquent que 22 % et 13 % des cellules HeLa transfectées respectivement avec le copolymère selon l'invention (R₁=R₂=H et m = 25 % et m = 4 %) expriment le transgène. Le nombre de cellules transfectées par la LPEI non modifiée est plus faible (environ 3 %).

### Exemple 3 : copolymère selon l'invention : R₁=R₂=H, m = 4% et complexe avec l'ADN d'un gène rapporteur codant la luciférase

On fait les mêmes synthèses de copolymère que dans l'exemple 1 ci-dessus, avec la seule différence que l'on effectue la modification chimique des motifs monomères de la LPEI à l'aide de groupements acide 3-amidopropionato-1-yl-(3-(3H-imidazol-4-yl)propanoique à un taux de seulement 4 %. Ce taux s'obtient en ne chauffant à reflux la LPEI au contact de l'agent modificateur que pour un jour. Dans les mêmes conditions que dans l'exemple 1, l'efficacité de la LPEI modifiée à 4 % (copolymère selon l'invention avec R₁= R₂= H et m= 4%) est d'environ 1.10⁷ RLU/mg de protéine (figure 5), à comparer avec une efficacité de 1,7.10⁶ RLU/mg de protéine pour la LPEI non modifiée (figure 1).

### Exemple 4 : copolymère selon l'invention : R₁=R₂=H, m = 66% et complexe avec l'ADN d'un gène rapporteur codant la luciférase

On fait les mêmes synthèses de copolymère que dans l'exemple 1 ci-dessus, avec la seule différence que l'on effectue la modification chimique des motifs monomères de la LPEI à l'aide de groupements acide 3-amidopropionato-1-yl-(3-(3H-imidazol-4-yl)propanoique à un taux de 66 %. Ce taux s'obtient en chauffant à reflux la LPEI au contact de l'agent modificateur pour une durée de 4 jours. Dans les mêmes conditions que dans l'exemple 1, l'efficacité de la LPEI modifiée à 66 % (copolymère selon l'invention avec R₁= R₂= H et m= 66%) est d'environ 1.10⁴ RLU/mg de protéine (Figure 5).

### Exemple 5 : copolymère selon l'invention : R₁= H, R₂=méthyle et m = 20% ou m = 66% et complexe avec l'ADN d'un gène rapporteur codant la luciférase

On fait les mêmes synthèses de copolymère que dans l'exemple 1 ci-dessus, avec la seule différence que l'on effectue la modification chimique des motifs monomères de la LPEI à l'aide de groupements 3-amidopropionato-1-yl-(3-(3H-imidazol-4-yl)propanoate méthyle ester à un taux de 20 %. La synthèse de l'acrylamide N-2-(3(3H-imidazol-4-yl)propanoate méthyle ester) (composé de formule IV avec R₁=H et R₂ = -CH3) s'effectue de la façon suivante : cet agent modificateur s'obtient dans les mêmes conditions que dans l'exemple 1, en remplaçant l'histidine par son ester méthylé : l'acrylamide N-2-(3(3H-imidazol-4-yl)propanoate méthyle ester). Le taux de 20 % s'obtient en chauffant à reflux la LPEI au contact de l'agent modificateur pour une durée de 2 jours. Dans les mêmes conditions que dans l'exemple 1, l'efficacité de la LPEI modifiée à 20 % (copolymère selon l'invention avec R₁=H, R₂=méthyle et m= 20 %) est d'environ 1,6.10⁸ RLU/mg de protéine, ce qui est comparable à celui obtenu à l'aide de l'histidine sous sa forme acide (exemple 1, figure 2 et figure 5 : His-LPEI à 25% et HisOMet-LPEI à 20 %). Par contre, la transfection obtenue à l'aide de LPEI modifiée à 66 % est très basse (copolymère selon l'invention R₁= R₂= méthyle et m= 66%), aux environs de 4.10³ RLU/mg de protéine (figure 5 : HisOMet-LPEI à 66%). Ce résultat est en accord avec le fait que cette LPEI trop fortement substituée par l'histidine ne condense pas fortement l'ADN (Figure 1).

Ainsi, par ces exemples, les inventeurs ont mis en évidence que les polyéthylènimines linéaires modifiées selon l'invention non seulement ont une efficacité de transfection supérieure à celle des polyéthylènimines correspondantes non modifiées, mais qu'elles ont également une toxicité moindre. L'avantage de cette modification ne réside pas seulement dans l'amélioration de l'efficacité de transfection, mais aussi dans le fait de garder une structure linéaire pour le squelette macromoléculaire et d'augmenter la solubilité dans l'eau du polymère ainsi modifié aux pH physiologiques.

Selon le taux de modification, l'efficacité et la toxicité varient, et cela en fonction de la nature des cellules transfectées.

## Revendications

1. Copolymère statistique de polyéthylènimine linéaire comprenant les deux unités monomères de formules I et II suivantes : 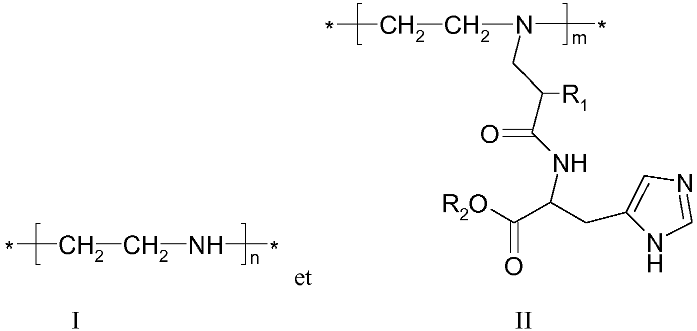 dans lesquels
R₁ représente un atome d'hydrogène ou un radical alkyle en C₁-C₆,
R₂ représente un atome d'hydrogène, un groupe alkyle en C₁-C₆, aryle ou aralkyle, dans lequel le groupe alkyle est en C₁-C₆,
n est un nombre compris entre 1 et 99 % des monomères totaux et
m est un nombre compris entre 1 et 99 % des monomères totaux, avantageusement entre 1 et 70 % des monomères totaux, plus avantageusement entre 5 et 35 % des monomères totaux,
avantageusement, m + n = 100 %.

2. Copolymère selon la revendication 1 **caractérisé en ce qu'**il comprend une unité monomère supplémentaire de formule III : 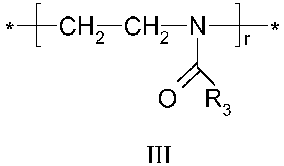 dans laquelle
R₃ représente un groupe alkyle en C₁-C₆ ;
r est un nombre compris entre 1 et 95 % des monomères totaux, avantageusement entre 1 et 10% des monomères totaux,
avantageusement, m + n + r= 100 %.

3. Procédé de préparation d'un copolymère selon l'une quelconque des revendications 1 ou 2, **caractérisé en ce qu'**il comprend l'étape de mise en contact d'une polyéthylènimine linéaire solubilisée dans de l'eau à un pH compris entre 6,5 et 7,5 avec le composé de formule IV suivante 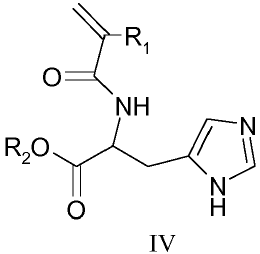 dans laquelle R₁ et R₂ sont tels que définis dans la revendication 1, avantageusement à la température de reflux de l'eau.

4. Procédé selon la revendication 3, **caractérisé en ce que** la polyéthylènimine linéaire a une masse molaire moyenne en nombre comprise entre 400 Da et 1 000 000 de Da, avantageusement entre 4000 et 40 000 Da, encore plus avantageusement entre 10 000 et 30 000 Da.

5. Procédé selon la revendication 3 ou 4, **caractérisé en ce que** le composé de formule générale IV est obtenu par réaction du composé de formule générale V suivante 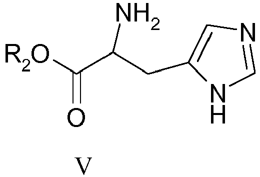 dans lequel R₂ est tel que défini dans la revendication 1 avec un composé de formule générale VI suivante 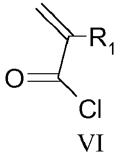 dans laquelle R₁ est tel que défini dans la revendication 1, avantageusement en milieu basique.

6. Composition pharmaceutique comprenant un copolymère selon l'une quelconque des revendications 1 ou 2 en tant que vecteur pharmaceutiquement acceptable et une substance active.

7. Utilisation du copolymère selon l'une quelconque des revendications 1 ou 2 en tant que vecteur pharmaceutiquement acceptable d'une substance active dans une composition pharmaceutique.

8. Complexe comprenant
a) le copolymère selon l'une quelconque des revendications 1 ou 2,
b) au moins une molécule anionique, avantageusement au moins un acide nucléique, en particulier choisi dans le groupe constitué par de l'ARN, de l'ADNc, de l'ADN génomique, de l'ADN plasmidique, de l'ADN antisens, de l'ARN messager, de l'ARN antisens, de l'ARN interférant, des ribozymes, de l'ARN de transfert, de l'ARN ribosomique, ou de l'ADN codant ces types d'ARN.

9. Complexe selon la revendication 8, dans lequel ledit acide nucléique comprend une séquence de gènes choisis parmi
a) des gènes marqueurs, avantageusement choisi parmi les gènes de :
- la luciférase de la luciole ;
- la protéine verte de méduse *Aequora Victoria ;*
- la béta-galactosidase d'E. Coli ;
- la chloramphénicol acétyle transférase ;
- résistance à un antibiotique, avantageusement de résistance à la néomycine ou à l'hygromycine,
b) des gènes à visée thérapeutique, avantageusement choisis parmi les gènes codants pour:
- des antigènes viraux, avantageusement la nucléoprotéine du virus de la grippe ou la protéine NEF ou GAG du virus VIH ;
- des antigènes associés aux tumeurs, avantageusement l'antigène MART1 des cancers à mélanome, les mucines ou l'antigène PSA des cancers de la prostate ;
- des antigènes bactériens ;
- des antigènes parasitaires et
c) des gènes à visée vaccinale avantageusement choisis parmi les gènes codant :
- la dystrophine et minidystrophine ;
- la protéine CFTR cystic fibrosis transmembrane conductance regulator associée à la mucoviscidose ;
- l'alpha1-antitrypsine ;
- l'Erythropoïétine ;
- les cytokines telles que les interleukines et le TNF facteur de nécrose des tumeurs) ;
- les facteurs de croissance tels que le TGFbéta et le PDGF ;
- les enzymes lysosomiques telles que la béta-glucosidase ;
- l'adénosine désaminase ;
- des ARN sens et antisens ;
- des ARN interférants ;
- des ribozymes ;
- les récepteurs des lipoprotéines de faible densité, déficient dans le cas d'hypercholestérolémie ;
- les facteurs de coagulation tels que les facteurs VII, VIII et IX ;
- la phénylalanine-hydroxylase ;
- la tyrosine hydroxylase ;
- la thymidine kinase du virus Herpes simplex ;
- les protéines du complexe majeur d'histocompatibilité, avantageusement les HLA-B7 ;
- la cytosine désaminase ,
et les éléments permettant son expression.

10. Composition pharmaceutique comprenant un complexe selon l'une quelconque des revendications 8 ou 9.

11. Procédé pour transférer *in vitro* un acide nucléique dans des cellules, ledit procédé comprenant la mise en contact desdites cellules avec au moins un complexe selon l'une quelconque des revendications 8 ou 9 ou une composition selon la revendication 10.

12. Procédé selon la revendication précédente **caractérisé en ce que** les cellules sont des cellules de mammifères, avantageusement choisies parmi :
- des cellules souches hématopoïétiques ;
- des cellules du système immunitaire, telles que des cellules dendritiques, des macrophages et des lymphocytes ;
- des cellules du foie ;
- des cellules des muscles squelettiques ;
- des cellules de la peau, telles que des fibroblastes, des kératinocytes, des cellules dendritiques et des mélanocytes ;
- des cellules des vaisseaux et microvaisseaux sanguins, telles que des cellules endothéliales et des cellules musculaires lisses ;
- des cellules épithéliales des voies aériennes ;
- des cellules du système nerveux central ;
- des cellules cancéreuses ;
- des cellules du tissu conjonctif, telles que des ténocytes.

13. Utilisation d'un copolymère selon l'une des revendications 1 ou 2 pour la préparation d'une composition pharmaceutique destinée à la délivrance intracellulaire de molécules anioniques, avantageusement d'acide nucléique.

14. Complexe selon l'une quelconque des revendications 8 ou 9 pour son utilisation en tant que médicament destiné à la délivrance intracellulaire d'acide nucléique.

15. Complexe selon l'une quelconque des revendications 8 ou 9, pour son utilisation en tant que médicament destiné au traitement de tumeurs, avantageusement de léiomyome utérin ou de tumeur maligne telle que le carcinome des ovaires, du sein ou de l'endomètre, pour la préparation d'un vaccin ou au traitement ou à la prévention de déficience métabolique congénitale ou acquise ou de maladies associées avec une expression génétique déficiente, avantageusement au traitement de maladies liées aux troubles d'un gène unique telles que les syndromes de déficit immunitaire combiné sévère, la mucoviscidose, l'hémophilie, la drépanocytose, la béta-thalassémie et la dystrophie musculaire.

## Patentansprüche

1. Lineares statistisches Polyethylenimin-Copolymer, umfassend die folgenden zwei Monomereinheiten der Formeln I und II: 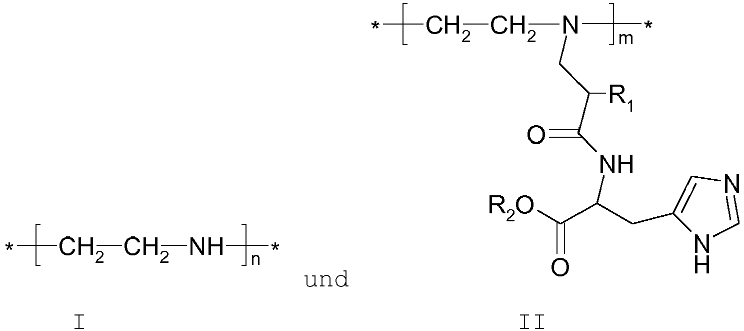 wobei
R₁ ein Wasserstoffatom oder einen Alkylrest aus C₁-C₆ darstellt,
R₂ ein Wasserstoffatom, eine Alkylgruppe aus C₁-C₆, Aryl oder Aralkyl darstellt, wobei die Alkylgruppe aus C₁-C₆ ist,
n eine Zahl zwischen 1 und 99 % der Gesamtmonomere ist und
m eine Zahl zwischen 1 und 99 % der Gesamtmonomere, vorteilhaft zwischen 1 und 70 % der Gesamtmonomere, vorteilhafter zwischen 5 und 35 % der Gesamtmonomere,
vorteilhaft m + n = 100 % ist.

2. Ein Copolymer nach Anspruch 1, **dadurch gekennzeichnet, dass** es eine zusätzliche Monomereinheit der Formel III umfasst: 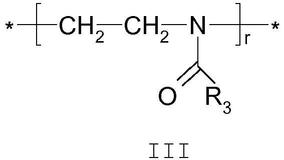 wobei
R₃ eine Alkylgruppe aus C₁-C₆ darstellt;
r eine Zahl zwischen 1 und 95 % der Gesamtmonomere, vorteilhaft zwischen 1 und 10 % der Gesamtmonomere ist,
vorteilhaft m + n + r = 100 %.

3. Verfahren zur Herstellung eines Copolymers nach einem beliebigen der vorstehenden Ansprüche 1 oder 2, **dadurch gekennzeichnet, dass** es den Schritt des Berührens eines linearen, in Wasser gelöstem Polyethylenimins mit einem pH-Wert zwischen 6,5 und 7,5 mit der Verbindung der folgenden Formel IV umfasst, 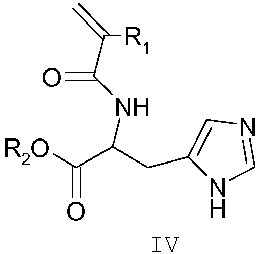 in der R₁ und R₂ wie in Anspruch 1 definiert sind, vorteilhafterweise bei der Rückflusstemperatur des Wassers.

4. Verfahren nach Anspruch 3, **dadurch gekennzeichnet, dass** das lineare Polyethylenimin eine zahlenmittlere Molmasse zwischen 400 Da und 1.000.000 Da, vorteilhaft zwischen 4.000 und 40.000 Da, noch vorteilhafter zwischen 10.000 und 30.000 Da aufweist.

5. Verfahren nach Anspruch 3 oder 4, **dadurch gekennzeichnet, dass** die Verbindung der allgemeinen Formel IV durch Reaktion der folgenden allgemeinen Formel V 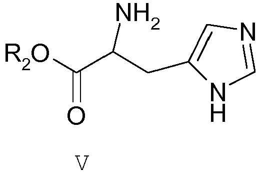 erzielt wird, wobei R₂ wie in Anspruch 1 definiert ist, mit einer Verbindung der folgenden allgemeinen Formel VI 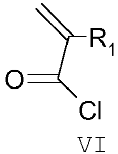 wobei R₁ wie in Anspruch 1 definiert ist, vorteilhaft in einem basischen Medium.

6. Pharmazeutische Zusammensetzung, umfassend ein Copolymer nach einem beliebigen der vorstehenden Ansprüche 1 oder 2 als pharmazeutisch verträglichen Träger und einen Wirkstoff.

7. Verwendung des Copolymers nach einem beliebigen der vorstehenden Ansprüche 1 oder 2 als pharmazeutisch verträglichen Träger eines Wirkstoffs in einer pharmazeutischen Zusammensetzung.

8. Komplex umfassend
a) das Copolymer nach einem beliebigen der vorstehenden Ansprüche 1 oder 2,
b) zumindest ein anionisches Molekül, vorteilhafterweise zumindest eine Nukleinsäure, insbesondere ausgewählt aus der Gruppe bestehend aus RNS, cDNS, genomischer DNS, Plasmid-DNS, Antisense-DNS, Messenger-RNS, Antisense-RNS, störender RNS, Ribozymen, Transfer-RNS, ribosomaler RNS oder DNS, die diese Arten von RNS codiert.

9. Komplex nach Anspruch 8, wobei die Nukleinsäure eine Sequenz von Genen umfasst, die unter
a) Markergenen ausgewählt sind, die vorteilhaft aus den Genen von:
- Glühwürmchen-Luciferase;
- grünes Protein der Qualle *Aequora victoria;*
- E. Coli Beta-Galaktosidase;
- Chloramphenicol-Acetyltransferase;
- Antibiotikaresistenz, vorteilhafterweise Neomycin- oder Hygromycinresistenz, ausgewählt sind, unter
b) Genen für therapeutische Zwecke, die vorteilhaft unter Genen ausgewählt sind, die für folgende codieren:
- Virusantigene, vorteilhaft das Nukleoprotein des Grippevirus oder das NEF- oder GAG-Protein des HIV-Virus;
- tumorassoziierte Antigene, vorteilhafterweise das MART1-Antigen von Melanom-Krebsen, die Mucine oder PSA-Antigene von Prostatakrebsen;
- bakterielle Antigene;
- Parasiten-Antigene und
c) Gene zu Impfzwecken, die vorteilhafterweise aus Genen ausgewählt sind, die für folgende codieren:
- Dystrophin und Minidystrophin;
- CFTR-Protein (cystic fibrosis transmembrane conductance regulator) mit Mukoviszidose verbunden;
- Alphal-Antitrypsin;
- Erythropoietin;
- Zytokine wie Interleukine und TNF Tumornekrosefaktor;
- Wachstumsfaktoren wie TGFbeta und PDGF;
- lysosomale Enzyme wie Beta-Glukosidase;
- Adenosindesaminase;
- Sense- und Antisense-RNS;
- störende RNS;
- Ribozyme;
- Lipoproteinrezeptoren mit niedriger Dichte, die bei Hypercholesterinämie unzureichend sind;
- Gerinnungsfaktoren wie die Faktoren VII, VIII und IX;
- Phenylalanin-Hydroxylase;
- Tyrosinhydroxylase;
- Thymidinkinase des Herpes simplex Virus;
- Proteine des großen Histokompatibilitätskomplexes, vorteilhaft HLA-B7;
- Cytosin Deaminase, und die Elemente für ihren Ausdruck.

10. Pharmazeutische Zusammensetzung, umfassend einen Komplex nach einem beliebigen der vorstehenden Ansprüche 8 oder 9.

11. Verfahren zum Übertragen *in vitro* einer Nukleinsäure in Zellen, wobei das Verfahren die Berührung der Zellen mit zumindest einem Komplex nach einem beliebigen der vorstehenden Ansprüche 8 oder 9 oder einer Zusammensetzung nach Anspruch 10 umfasst.

12. Verfahren nach dem vorstehenden Anspruch, **dadurch gekennzeichnet, dass** die Zellen Säugetierzellen sind, die vorteilhaft ausgewählt sind unter:
- hämatopoietischen Stammzellen;
- Immunsystemzellen, wie dendritische Zellen, Makrophagen und Lymphozyten;
- Leberzellen;
- Skelettmuskelzellen;
- Hautzellen, wie Fibroblaste, Keratinozyten, dendritische Zellen und Melanozyten;
- Blutgefäß- und mikrovaskuläre Zellen, wie Endothelzellen und glatte Muskelzellen;
- Epithelzellen der Atemwege;
- Zellen des zentralen Nervensystems;
- Krebszellen;
- Zellen des Bindegewebes, wie z.B. Tenozyten.

13. Verwendung eines Copolymers nach einem beliebigen der vorstehenden Ansprüche 1 oder 2 zur Herstellung einer pharmazeutischen Zusammensetzung für die intrazelluläre Abgabe von anionischen Molekülen, vorteilhaft Nukleinsäure.

14. Komplex nach einem beliebigen der vorstehenden Ansprüche 8 oder 9 zur Verwendung als Arzneimittel zur intrazellulären Abgabe von Nukleinsäure.

15. Komplex nach einem beliebigen der vorstehenden Ansprüche 8 oder 9 zur Verwendung als Arzneimittel zur Behandlung von Tumoren, vorteilhafterweise uterines Leiomyom oder bösartigen Tumor wie Eierstock-, Brust- oder Endometriumkarzinom, zur Herstellung eines Impfstoffs oder zur Behandlung oder Prävention von angeborenem oder erworbenem Stoffwechselmangel oder Krankheiten, die mit einer mangelhaften Genexpression verbunden sind, vorteilhafterweise für die Behandlung von Krankheiten im Zusammenhang mit einzelnen Genstörungen wie schweren kombinierten Immundefizienzsyndromen, Mukoviszidose, Hämophilie, Sichelzellkrankheit, Beta-Thalassämie und Muskeldystrophie.

## Claims

1. A random copolymer of linear polyethylenimine comprising the two monomeric units of the following formulae I and II: 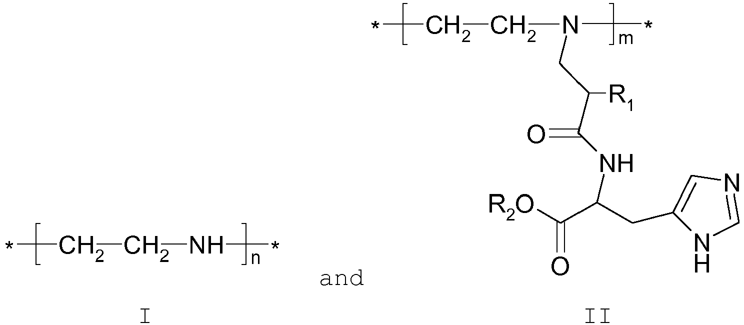 wherein
R₁ represents a hydrogen atom or a C₁-C₆ alkyl group,
R₂ represents a hydrogen atom, a C₁-C₆ alkyl, aryl or aralkyl group, in which the alkyl group is a C₁-C₆ alkyl,
n is a number comprised between 1 and 99% of the total monomers and
m is a number comprised between 1 and 99% of the total monomers, advantageously between 1 and 70% of the total monomers, more advantageously between 5 and 35% of the total monomers, advantageously m + n = 100%.

2. The copolymer according to claim 1, **characterized in that** it comprises an additional monomer unit of formula III: 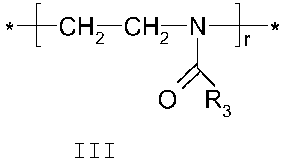 wherein
R₃ represents a C₁-C₆ alkyl group;
r is a number comprised between 1 and 95% of the total monomers, advantageously between 1 and 10% of the total monomers, advantageously m + n + r = 100%.

3. A method for preparing a copolymer according to any one of claim 1 or 2, **characterized in that** it comprises the step for putting a linear polyethylenimine solubilized in water at a pH comprised between 6.5 and 7.5 in contact with the compound of the following formula IV: 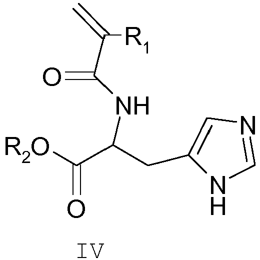 wherein R₁ and R₂ are as defined in claim 1, advantageously at water reflux temperature.

4. The method according to claim 3, **characterized in that** the linear polyethylenimine has a number average molar mass comprised between 400 Da and 1,000,000 Da, advantageously between 4,000 Da and 40,000 Da, more advantageously between 10,000 Da and 30,000 Da.

5. The method according to claim 3 or 4, **characterized in that** the compound of general formula IV is obtained by reacting the compound of the following general formula V 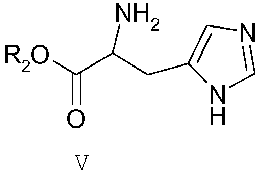 wherein R₂ is as defined in claim 1, with a compound of the following general formula VI 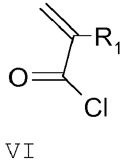 wherein R₁ is as defined in claim 1, advantageously in a basic medium.

6. A pharmaceutical composition comprising a copolymer according to any one of claims 1 or 2 as a pharmaceutically acceptable vector and an active substance.

7. Use of a copolymer according to any one of claims 1 or 2 as a pharmaceutically acceptable vector of an active substance in a pharmaceutical composition.

8. A complex comprising:
a) the copolymer according to any one of claims 1 or 2,
b) at least one anionic molecule, advantageously at least one nucleic acid in particular selected from the group formed by RNA, cDNA, genomic DNA, plasmid DNA, antisense DNA, messenger RNA, antisense RNA, interfering RNA, ribozymes, transfer RNA, ribosomal RNA, or DNA coding for these RNA types.

9. The complex according to claim 8, wherein said nucleic acid comprises a sequence of genes selected from
a) marker genes, advantageously selected from the genes of:
- luciferase of fireflies;
- green protein of *Aequora Victoria* jellyfishes;
- beta-galactosidase of *E.coli;*
- chloramphenicol acetyl transferase;
- resistance to an antibiotic, advantageously resistance to neomycin or hygromycin,
b) genes with therapeutic purpose, advantageously selected from the genes coding for:
- viral antigens, advantageously the nucleoprotein of the influenza virus or the NEF or GAG protein of the HIV virus;
- antigens associated with tumors, advantageously the antigen MART1 of melanoma cancer, mucins or PSA antigen of prostate cancers;
- bacterial antigens;
- parasite antigens, and
c) genes with vaccinal purpose, advantageously selected from the genes coding for:
- dystrophin and minidystrophin;
- cystic fibrosis transmembrane conductance regulator protein (CFTR) associated with cystic fibrosis;
- alphal-antitrypsin;
- erythropoietin;
- cytokines such as interleukins and tumor necrosis factor (TNF);
- growth factors such as TGFbeta and PDGF;
- lysosomal enzymes such as beta-glucosidase;
- adenosine deaminase;
- sense and anti-sense RNAs;
- interfering RNAs;
- ribozymes;
- receptors of low density lipoproteins, lacking in the case of hypercholesterolemia;
- coagulation factors such as the factors VII, VIII and IX;
- phenylalanine-hydroxidase;
- tyrosine hydroxylase;
- thymidine kinase of the Herpes simplex virus;
- proteins of the major histocompatibility complex, advantageously the HLA-B7;
- cytosine deaminase,
and elements allowing its expression.

10. A pharmaceutical composition comprising a complex according to any one of claims 8 or 9.

11. A method for transferring *in vitro* a nucleic acid into cells, said method comprising the putting of said cells in contact with at least one complex according to any one of claims 8 or 9 or a composition according to claim 10.

12. The method according to the preceding claim, **characterized in that** the cells are mammal cells, advantageously selected from:
- hematopoietic stem cells;
- cells of the immune system, such as dendritic cells, macrophages and lymphocytes;
- liver cells;
- cells of skeletal muscles;
- skin cells, such as fibroblasts, keratinocytes, dendritic cells, melanocytes;
- cells of blood vessels and microvessels, such as endothelial cells and smooth muscle cells;
- epithelial cells of airways;
- cells of the central nervous system;
- cancer cells;
- cells of the connective tissue, such as tenocytes.

13. Use of a copolymer according to any of claims 1 or 2 for preparing a pharmaceutical composition for intracellular delivery of anionic molecules, advantageously of nucleic acid.

14. The complex according to any one of claims 8 or 9, for its use as a medicine for intracellular delivery of nucleic acid.

15. The complex according to any one of claims 8 or 9, for its use as a medicine for treating tumors, advantageously uterine leiomyoma or malignant tumor such as carcinoma of the ovaries, the breast or the endometrium, for preparing a vaccine or for treating or preventing congenital or acquired metabolic deficiency or diseases associated with deficient genetic expression, advantageously for treating diseases associated with single gene disorders such as severe combined immune deficit syndromes, cystic fibrosis, hemophilia, drepanocytosis, beta-thalassemia and muscular dystrophy.
